(19) Europäiscnes Patentamt
European Patent Office
Office européen des brevets

(11) Publication number: **0 100 158**
**A2**

# (12) EUROPEAN PATENT APPLICATION

(21) Application number: **83303851.6**

(22) Date of filing: **01.07.83**

(51) Int. Cl.³: **C 07 D 213/74**, C 07 D 213/70, C 07 D 213/65

(30) Priority: **28.07.82 US 402514**

(43) Date of publication of application: **08.02.84 Bulletin 84/6**

(84) Designated Contracting States: **BE CH DE FR GB IT LI NL SE**

(71) Applicant: **THE UPJOHN COMPANY, 301 Henrietta Street, Kalamazoo, Michigan 49001 (US)**

(72) Inventor: **Lin, Chiu-Hong c/o THE UPJOHN COMPANY, 301 Henrietta Street, Kalamazoo Michigan 49001 (US)**

(74) Representative: **Perry, Robert Edward et al, GILL JENNINGS & EVERY 53-64 Chancery Lane, London WC2A 1HN (GB)**

(54) **(3-pyridinyl)heteroalkarylalkanols, alkanoic acids and esters.**

(57) Compounds of the formula

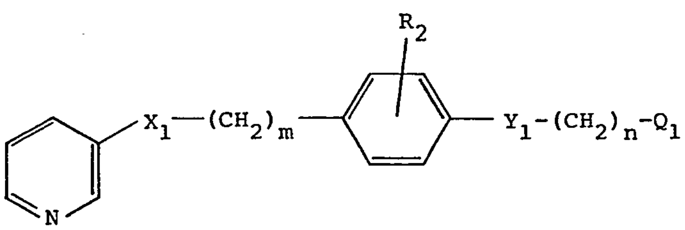

I

wherein $X_1$ and $Y_1$ are heteroatoms (or $Y_1$ is a bond), $R_2$ is hydrogen or a ring substituent, $Q_1$ is $CH_2QH$, OH, SH, $NHR_3$, $N(R_3)_2$ (the $R_3$'s are independently selected from H, $C_{1-5}$ alkyl and CHO) or optionally esterified or salified carboxyl, and m and n are each up to 5 (n can be zero). These compounds can be thromboxane $A_2$ synthetase inhibitors.

EP 0 100 158 A2

# (3-PYRIDINYL)HETEROALKARYLALKANOLS, ALKANOIC ACIDS AND ESTERS

The present invention relates to 3-pyridinyl-ω-carboxylic acids containing oxa, aza, thia and phenylene groups in the side chain. Such compounds are potent thromboxane $A_2$ inhibitors and as such represent useful pharmacological agents.

Since the discovery that human platelets convert the prostaglandin endoperoxide ($PGH_2$) into a labile proaggregatory molecule known as thromboxane $A_2$ ($TXA_2$), researchers have sought compounds that could selectively inhibit the biological activity of $TXA_2$. This end may be achieved in two different ways: the synthesis of $TXA_2$ can be blocked by inhibiting the $TXA_2$ synthetase, or a compound could be a receptor level antagonist of $TXA_2$. As therapeutic agents, $TXA_2$ synthetase inhibitors are more useful. See, e.g., R. Gorman, "Biological and Pharmacological Evaluation of Thomboxane Synthetase Inhibitors," Advances in Prostaglandin and Thromboxane Research, 6:417 (1980), and references cited therein. Most important are compounds which selectively inhibit $TXA_2$ synthetase.

A number of $TXA_2$ synthetase inhibitors are known. See for example the bi-heterocyclic 9,11-trideoxy-PGF-type compounds disclosed in US-A-4,112,224; SQ 80,388 [1-(3-phenyl-2-propenyl)-1H-imidazole] disclosed in D. Harris, et al., Advances in Prostaglandin and Thromboxane Research 6:437 (1980); pyridine and its derivatives, disclosed in T. Miyamoto, et al., Advances in Prostaglandin and Thromoboxane Research, 6:443 (1980), and GB-A-2,039,903 (abstracted in Derwent Farmdoc No. 50111C (1980)). See also H.Tai, et al., Advances in Prostaglandin and Thromboxane Research, 6:447 (1980). Other compounds which have been disclosed as thromboxane synthetase inhibitors, include sodium p-benzyl-4(1-oxo-2-(4-chlorobenzyl)-3-phenylpropyl)phenyl phosphate, imidazoles, nordihydroguaiaretic acid, and 12L-hydroperoxy-5,8,10,14-eicosa-tetraenoic acid (HETE). As noted in the above British patent specification, however, the inhibitory activity of these latter compounds on thromboxane synthetase is very weak, making them unsatisfactory as practically effective medicines.

French patent 1,585,085 (Derwent Farmdoc 40389R) discloses certain N-pyridinyl/anthranilic acids which are stated to be useful as analgesic, antipyretic, anti-inflammatory and anti-rheumatic agents. Japanese Kokai 75 111,076 (Derwent Farmdoc 1024X) discloses certain pyridyloxy-phenylalkanoic or pyridyloxy-benzoic acid derivatives which are stated to be useful as anti-inflammatory, anti-rheumatic and analgesic agents. European Patent 176 (Derwent Farmdoc 02309B) discloses certain pyridyloxy-phenoxy-alkanoic acid derivatives which are stated to be useful as herbicides and plant growth regulators. Denny, et al., J. Med. Chem. 20:1242 (1977) discloses pyridinyl anthranilic acids.

Certain 2-pyridinyl-phenylene compounds are disclosed in Derwent Farmdoc nos. 20536F; 29402F; 11056T; 06401A; 11911B; 12380B; and 50291C; and C.A. 86:17135U. Other pyridinyl-phenylene compounds are disclosed in Derwent Farmdoc Nos. 75975R; 75002U; and 03847D.

## SUMMARY OF THE INVENTION

Surprisingly and unexpectedly it has been found that selective thromboxane synthetase inhibition may be achieved by employing a compound of the formula I or a pharmacologically acceptable acid addition salt thereof,

wherein $X_1$ is
- (a) -O-,
- (b) -S-, or
- (c) $-NR_3-$;

wherein $Y_1$ is
- (a) -O-,
- (b) -S-,
- (c) $-NR_3-$, or
- (d) a valence bond;

wherein $R_2$ is
- (a) hydrogen,
- (b) hydroxy,
- (c) methoxy,
- (d) acetoxy,
- (e) fluoro,
- (f) chloro,
- (g) bromo,
- (h) methyl,

(i) trifluoromethyl,

(j) dimethylamino, or

(k) nitro;

wherein $Q_1$ is

(a) $-CO_2R_1$,

(b) $-CH_2OH$,

(c) -OH,

(d) -SH, or

(e) $-NR_3$;

wherein $R_1$ is

(a) hydrogen,

(b) a pharmacologically acceptable cation,

(c) $(C_1-C_{12})$ alkyl,

(d) $(C_3-C_{10})$ cycloalkyl,

(e) $(C_7-C_{12})$ aralkyl,

(f) phenyl,

(g) phenyl mono-, di-, or trisubstituted by chloro, or $(C_1-C_3)$ or alkyl, or

(h) phenyl para-substituted by

(1) $-NHCO-R_{25}$,

(2) $-O-CO-R_{26}$,

(3) $-CO-R_{24}$,

(4) $-O-CO-(p-Ph)-R_{27}$, or

(5) $-CH=N-NH-CO-NH_2$,

wherein $R_{24}$ is phenyl or acetamidophenyl, $R_{25}$ is methyl, phenyl, acetamidophenyl, benzamidophenyl, or amino, $R_{26}$ is methyl, phenyl, amino or methoxy, $R_{27}$ is hydrogen or acetamido, and -(p-Ph) is 1,4-phenylene;

wherein $R_3$ is

(a) hydrogen

(b) $(C_1-C_5)$alkyl, or

(c) -CHO;

wherein m is an integer from one to 5;

wherein n is an integer from zero to 5, with the proviso that n is zero only when $Y_1$ is a valence bond and $Q_1$ is $-CO_2R_1$.

The carbon atom content of various hydrocarbon-containing moieties is indicated by a prefix designating the minimum and maximum number of carbon atoms in the moiety, i.e., the prefix $(C_i-C_j)$

indicates a moiety of the integer "i" to the integer "j" carbon atoms, inclusive. Thus $(C_1-C_3)$alkyl refers to alkyl of one to 3 carbon atoms, inclusive, or methyl, ethyl, propyl, and isopropyl.

Examples of alkyl of one to 12 carbon atoms, inclusive, are methyl, ethyl, propyl, butyl, pentyl, hexyl, heptyl, octyl, nonyl, decyl, undecyl, dodecyl, and isomeric forms thereof.

Examples of cycloalkyl of 3 to 10 carbon atoms, inclusive, which includes alkyl-substituted cycloalkyl, are cyclopropyl, 2-methylcyclopropyl, 2,2-dimethylcyclopropyl, 2,3-diethylcyclopropyl, 2-butylcyclopropyl, cyclobutyl, 2-methylcyclobutyl, 3-propylcyclobutyl, 2,3,4-triethylcyclobutyl, cyclopentyl, 2,2-dimethylcyclopentyl, 2-pentylcyclopentyl, 3-tert-butylcyclopentyl, cyclohexyl, 4-tert-butylcyclohexyl, 3-isopropylcyclohexyl, 2,2-dimethylcyclohexyl, cycloheptyl, cyclooctyl, cyclononyl and cyclodecyl.

Examples of aralkyl of 7 to 12 carbon atoms, inclusive, are benzyl, 2-phenethyl, 1-phenylethyl, 2-phenylpropyl, 4-phenylbutyl, 3-phenylbutyl, 2-(1-naphthylethyl), and 1-(2-naphthylmethyl).

Examples of phenyl substituted by one to 3 chloro or alkyl of one to 3 carbon atoms, inclusive, are p-chlorophenyl, m-chlorophenyl, 2,4-dichlorophenyl, 2,4,6-trichlorophenyl, p-tolyl, m-tolyl, o-tolyl, p-ethylphenyl, 2,5-dimethylphenyl, 4-chloro-2-methylphenyl, and 2,4-dichloro-3-methylphenyl.

The compounds of the present invention may be in the form of pharmacologically acceptable salts. These salts are formed when $R_1$ is a pharmacologically acceptable cation. Such cations include: pharmacologically acceptable metal cations, ammonium, amine cations, or quaternary ammonium cations.

Especially preferred metal cations are those derived from the alkali metals, e.g., lithium, sodium, and potassium, and from the alkaline earth metals, e.g., magnesium and calcium, although cationic forms of other metals, e.g., aluminum, zinc, and iron are within the scope of this invention.

Pharmacologically acceptable amine cations are those derived from primary, secondary, or tertiary amines. Examples of suitable amines are methylamine, dimethylamine, trimethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethyl-

enediamine, diethylenetriamine, and the like aliphatic, cycloaliphatic, araliphatic amines containing up to and including about 18 carbon atoms, as well as heterocyclic amines, e.g., piperidine, morpholine, pyrrolidine, piperazine, and lower-alkyl derivatives thereof, e.g.,

1-methylpiperidine,
4-ethylmorpholine,
1-isopropylpyrrolidine,
2-methylpyrrolidine,
1,4-dimethylpiperazine,
2-methylpiperidine,

and the like, as well as amines containing water-solubilizing or hydrophilic groups, e.g,

mono-, di-, and triethanolamine,
ethyldiethanolamine,
N-butylethanolamine,
2-amino-1-butanol,
2-amino-2-ethyl-1,3-propanediol,
2-amino-2-methyl-1-propanol,
tris(hydroxymethyl)aminomethane,
N-phenylethanolamine,
N-(p-tert-amylphenyl)diethanolamine,
glactamine,
N-methylglycamine,
N-methylglucosamine,
ephedrine,
phenylephrine,
epinephrine,
procaine,

and the like. Further useful amine salts are the basic amino acid salts, e.g.,

lysine and
arginine.

Examples of suitable pharmacologically acceptable quaternary ammonium cations are

tetramethylammonium,
tetraethylammonium,
benzyltrimethylammonium,
phenyltriethylammonium, and the like.

Pharmaceutically acceptable acid addition salts are formed at the heterocyclic amine moiety and are also useful for administering the compounds of this invention. These salts include hydrochloride, hydrobromide, hydroiodide, sulfate, phosphate, acetate, propionate, lactate, maleate, malate, succinate, tartrate, and the like. They are prepared by methods well known in the art.

The compounds of the present invention will be named herein using the Chemical Abstracts numbering system (see Naming and Indexing of Chemical Substances for Chemical Abstracts during the Ninth Collective Period (1972-1976), a reprint of section IV from the Volume 76 Index Guide.)

The compounds of the present invention were tested for $TXA_2$ inhibition as follows:

Rabbit aortic strips were superfused in series with Krebs solution. Thromboxane $A_2$ ($TXA_2$) was generated by mixing prostaglandin $H_2$ ($PGH_2$) with human platelet microsomes (HPM).

Potential inhibitors were tested by comparing the response of the rabbit aorta to the amount of $TXA_2$ produced by mixing $PGH_2$ and HPM without the test compound in the reaction medium and then the amount of $TXA_2$ produced when the test compound was added to the HPM 5 minutes before the HPM was mixed with $PGH_2$. By this means compounds which selectively inhibit $TXA_2$ synthetase are found. For a discussion of $TXA_2$ synthetase inhibition testing see, e.g., R. Gorman, supra.

Using this test system, three compounds, sodium 2-hydroxy-5-[N-(3-pyridinyl)aminomethyl]benzoate, (Example 8); sodium 2-methoxy-5-[N-(3-pyridinyl)aminomethyl]-benzoate (Example 6); and 4-[[(3-pyridinyl-amino)methyl]phenoxy]-acetic acid, sodium salt (Example 4) have been shown to be the most effective in inhibiting $TXA_2$ formation. These compounds have approximate $ED_{50}$'s in this system of 10 ng/ml, between 10 and 100 ng/ml, and between 10 and 1000 ng/ml, respectively.

The novel compounds of this invention have thus been shown to be highly active as selective inhibitors of the thromboxane synthetase enzyme system. Accordingly, these novel compounds are useful for administration to mammals, including humans, whenever it is desirable medically to inhibit this enzyme system. For a discussion of the utility of $TXA_2$ inhibitors, see, e.g., Derwent Farmdoc Nos. 18399B; 72896B; 72897B; 63409B; 03755C; 03768C; and 50111C.

Thus, for example, these novel compounds are useful as anti-inflammatory agents in mammals and especially humans, and for this purpose, are administered systemically and preferably orally. For oral administration, a dose range of 0.05 to 50 mg per kg of human body weight is used to give relief from pain associated with inflammatory disorders such as rheumatoid arthritis. They are also administered intravenously in aggravated cases of inflammation, preferably in a dose range 0.01 to 100 μg per kg per minute until relief from pain is attained. When used for these purposes, these novel compounds cause fewer and lesser undesirable side effects than do the known synthetase inhibitors used to treat inflammation, for example, aspirin and indomethacin. When these novel compounds are administered orally, they are formulated as tablets, capsules, or as liquid preparations, with the usual pharmaceutical carriers, binders, and the like. For intravenous use, sterile isotonic solutions are preferred.

These compounds are useful whenever it is desired to inhibit platelet aggregation, reduce the adhesive character of platelets, and remove or prevent the formation of thrombi in mammals, including man, rabbits, dogs, and rats. For example, these compounds are useful in the treatment and prevention of myocardial infarcts, to treat and prevent post-operative thrombosis, to promote patency of vascular grafts following surgery, and to treat conditions such as atherosclerosis, arteriosclerosis, blood clotting defects due to lipemia, and other clinical conditions in which the underlying etiology is associated with lipid imbalance or hyperlipidemia. For these purposes, these compounds are administered systemically, e.g., intravenously, subcutaneously, intramuscularly, and in the form of sterile implants for prolonged action. For rapid response especially in emergency situations, the intravenous route of administration is preferred. Doses in the range about 0.005 to about 20 mg per kg of body weight per day are used, the exact dose depending on the age, weight, and condition of the patient or animal, and on the frequency and route of administration.

These compounds are further useful as additives to blood, blood products, blood substitutes, or other fluids which are used in artificial extracorporeal circulation or perfusion of isolated body portions, e.g., limbs and organs, whether attached to the original body, detached and being preserved or prepared for transplant, or

attached to a new body. During these circulations and perfusions, aggregated platelets tend to block the blood vessels and portions of the circulation apparatus. This blocking is avoided by the presence of these compounds. For this purpose, the compound is added gradually or in single or multiple portions to the circulating blood, to the blood of the donor animal, to the perfused body portion, attached or detached, to the recipient, or to two or all of these at a total steady state dose of about 0.001 to 10 mg per liter of circulating fluid. It is especially useful to use these compounds in laboratory animals, e.g., cats, dogs, rabbits, monkeys, and rats, for these purposes in order to develop new methods and techniques for organ and limb transplants.

The compounds of the present invention are useful in mammals, including humans and certain useful animals, e.g., dogs and pigs, to reduce or avoid gastrointestinal ulcer formation, and accelerate the healing of such ulcers already present in the gastrointestinal tract. For this purpose, these compounds are injected or infused intravenously, subcutaneously, or intramuscularly in an infusion dose range about 0.1 μg to about 500 μg/kg of body weight per minute, or in a total daily dose by injection or infusion in the range about 0.1 to about 20 mg/kg of body weight per day, the exact dose depending on the age, weight, and condition of the patient or animal, and on the frequency and route of administration.

The novel compounds are used for the purposes described above in the free acid form, in ester form, and in the pharmacologically acceptable salt form. When the ester form is used, the ester is any of those within the above definition of $R_1$. However, it is preferred that the ester be alkyl of one to 12 carbon atoms, inclusive. Of the alkyl esters, methyl and ethyl are especially preferred for optimum absorption of the compound by the body or experimental animal system; and straight-chain octyl, nonyl, decyl, undecyl, and dodecyl are especially preferred for prolonged activity in the body or experimental animal.

Thromboxane synthetase converts $PGH_2$ (prostaglandin endoperoxide) into $TXA_2$. $PGH_2$ is also converted to prostacyclin, $PGD_2$, and other compounds by other enzymes. Thus, because the compounds of this invention inhibit thromboxane $A_2$ synthetase, they increase the $PGH_2$ substrate and thus increase the amount of endogenous prostacyclin.

Therefore, they are also useful for many of the pharmacological purposes for which prostacyclin is employed.

Prostacyclin and a thromboxane synthetase inhibitor have both been shown to be effective in controlling tumor cell metastasis, see, e.g., K. Honn, et al., "Thromboxane Synthetase Inhibitors and Prostacyclin Can Control Tumor Cell Metastasis," an Abstract of the Twentieth Annual Meeting of the American Society for Cell Biology, in the Journal of Cell Biology, 87:64 (1980).

Similarly, prostacyclin has been shown to be an effective antihypertensive agent. The compounds of the present invention are also used for this purpose. (See, e.g., British patent specification 2,039,903A).

For a general discussion of the utility of $TXA_2$ synthetase inhibitors which increase endogenous prostacyclin, see, Aiken, et al. J. Pharmacol. Exp. Ther., 219:299 (1981).

The compounds of the present invention are prepared by the methods depicted in Charts A-D.

In Chart A, 3-aminopyridine of the formula A-1 (a well known and readily available compound) is thermally condensed with an appropriate aldehyde of the formula A-2 to yield the formula A-3 imine which is reduced with sodium borohydride to yield the formula A-4 product. This is described more fully in Example 1. This chart also depicts a method for preparing compounds of the present invention wherein $X_1$ is -O-, -S-, or $-NR_3-$, $Y_1$ is a valence bond, and n is zero. An appropriate aldehyde of the formula A-2 is reduced with sodium borohydride to yield the formula A-6 compound. An alcohol of the formula A-6 is converted to a tosylate or halide (chloride, bromide, or iodide) of the formula A-7 (wherein $X_2$ is chloro, fluoro, bromo, or -O-tosylate) by the methods well known in the art. Alkylation of the formula A-7 compound with anions generated from 3-hydroxypyridine (Aldrich Chemical Co.) 3-mercaptopyridine [H. Fuerst, et al., J. Prakt. Chem. 36, 160 (1967)], or 3-aminopyridine (Aldrich Chemical Co.), utilizing sodium hydride or potassium carbonate as the base and dimethylformamide (DMF), glyme, or acetone as the solvent, gives the formula A-8 product. Corresponding pharmaclogically acceptable salts may be prepared by means well known in the art, e.g., treatment with sodium hydroxide in methanol, to yield the formula A-5 or A-9 compound wherein $R_1$ is sodium.

Chart B depicts a method for preparing compounds of the present invention wherein $Y_1$ is -O- and $X_1$ is -O-, -S-, or $-NR_3-$. An alcohol of the formula B-1 is reacted with an alkyl halide of the formula $X_3-(CH_2)_n-CO_2CH_3$ (wherein $X_3$ is chloro, bromo, or iodo), in the presence of an appropriate base and solvent, e.g., potassium carbonate and glyme, to yield the formula B-2 aldehyde-ester. This is described more fully in Preparation 1. This formula B-2 aldehyde-ester is then condensed with 3-aminopyridine to yield the corresponding imine of the formula B-3. This imine of the formula B-3 is reduced with sodium borohydride to yield the compounds of the invention corresponding to formulas B-4 and B-5. The aldehyde-ester of the formula B-2 is reduced with sodium borohydride to yield the formula B-6 compound. An alcohol of the formula B-6 compound is converted to the tosylate or halide (chloride, bromide, or iodide) of the formula B-7 by methods well known in the art. Alkylation of the formula B-7 compound (as described above regarding conversion of the formula A-7 compound to the formula A-8 compound), gives the formula B-8 product. The corresponding pharmacologically acceptable salts may be prepared as described above.

Chart C depicts a method for preparing compounds of the present invention wherein $Y_1$ is -S- and $X_1$ is -O-, -S-, or $-NR_3-$. An alcohol of the formula C-1 is protected by an alcohol protecting group of the formula $R_{18}$ such as a tetrahydropyranyl, a benzyl or a dimethyl-t-butylsilyl group by means well known in the art, to give the formula C-2 compound. This methyl-thio aryl compound of the formula C-2 is converted into an aryl thiol compound of the formula C-3 by the methods known in the art [see, e.g., J. M. Lavanish, Tetrahedron Lett. 3847 (1973)]. Alkylation of this formula C-3 compound with an alkyl halide of the formula $X_3-(CH_2)_n-CO_2CH_3$ (wherein $X_3$ is chloro, bromo, or iodo) in the presence of an appropriate base and solvent, e.g., potassium carbonate and glyme, yields the ester of the formula C-4. Removal of the alcohol protecting group yields the alcohol of the formula C-5. Conversion of this alcohol into a tosylate or halide followed by alkylation, as described above regarding the conversion of (from A-6 to A-7 to A-8), yields the compound of the formula C-7 wherein $X_1$ is -O-, -S-, or $-NR_3-$. The corresponding pharmacologically acceptable salts may be prepared as described above.

Chart D depicts a method for preparing compounds of the present

invention wherein $Y_1$ is $-NR_3-$ and $X_1$ is -O-, -S-, or $-NR_3-$. An alcohol of the formula D-1 is protected by an alcohol protecting group, as described above, to give the compound of the formula D-2. Alkylation (D-2 to D-3), deprotection of the alcohol protecting group (D-3 to D-4), conversion of the alcohol to the tosylate or halide (D-4 to D-5) and alkylation (D-5 to D-6) as described above, give the compound of the formula D-6 wherein $X_1$ is -O-, -S-, or $-NR_3-$. The corresponding pharmacologically acceptable salts may be prepared as described above.

In the Charts $R_{18}$ is a silyl protecting group of the formula $-Si(G_1)_3$. $G_1$ is alkyl of one to 4 carbon atoms, cycloalkyl of 3 to 10 carbon atoms, inclusive, aralkyl of 7 to 12 carbon atoms, inclusive, phenyl, or phenyl substituted with one or 2 fluoro, chloro, or alkyl of one to 4 carbon atoms, with the proviso that in a $-Si(G_1)_3$ moiety the various $G_1$'s are the same or different and at least one $G_1$ is hindered (such as tert-butyl). Silyl groups within the scope of $-Si(G_1)_3$ include dimethylphenylsilyl, triphenylsilyl, t-butyldimethylsilyl, or methylphenylbenzylsilyl. With regard to $G_1$, examples of alkyl are methyl, ethyl, propyl, isobutyl, butyl, sec-butyl, tert-butyl, pentyl, and the like. Examples of aralkyl are benzyl, phenethyl, α-phenylethyl, 3-phenylpropyl, α-maphthylmethyl, and 2-(α-maphthyl)ethyl. Examples of phenyl substituted with halo or alkyl are p-chlorophenyl, m-fluorophenyl, o-tolyl, 2,4-dichlorophenyl, p-tert-butylphenyl, 4-chloro-2-methylphenyl, and 2,4-dichloro-3-methylphenyl. Tert-butyldimethylsilyl is most preferred as a silylating agent.

These silyl groups are known in the art. See for example, Pierce "Silylating of Organic Compounds," Pierce Chemical Company, Rockford Ill. (1968). When silylated products of the charts below are intended to be subjected to chromatographic purification, then the use of silyl groups known to be unstable to chromatography is to be avoided. Further, when silyl groups are to be introduced selectively, silylating agents which are readily available and known to be useful in selective silylations are employed. For example, triphenylsilyl and t-butyldimethylsilyl groups are employed when selective introduction is required. A particularly useful silyl group for this purpose is t-butyldimethylsilyl, although other silyl groups are likewise employed.

Other protective groups within the scope of $R_{18}$ are any group which replaces a hydroxy hydrogen and is neither attacked by nor is as reactive to the reagents used in the transformations used herein as a hydroxy is and which is subsequently replaceable by acid hydrolysis with hydrogen. Several such protective groups are known in the art, e.g., tetrahydropyranyl and substituted tetrahydropyranyl. See for reference E.J. Corey, Proceedings of the Robert A. Welch Foundation Conferences on Chemical Research, XII Organic Synthesis, pgs. 51-79 (1969). Those blocking groups which have been found useful include:

(a) tetrahydropyranyl;

(b) tetrahydrofuranyl; and

(c) a group of the formula $-C(OR_{11})(R_{12})-CH(R_{13})(R_{14})$, wherein $R_{11}$ is alkyl of one to 18 carbon atoms, inclusive, cycloalkyl of 3 to 10 carbon atoms, inclusive, aralkyl of 7 to 12 carbon atoms, inclusive, phenyl or phenyl substituted wityh one to 3 alkyl of one to 4 carbon atoms, inclusive, wherein $R_{12}$ and $R_{13}$ are alkyl of one to 4 carbon atoms, inclusive, phenyl, phenyl substituted with one, 2, or 3 alkyl of one to 4 carbon atoms, inclusive, or when $R_{12}$ and $R_{13}$ are taken together $-(CH_2)_a-$ or when $R_{12}$ and $R_{13}$ are taken together $-(CH_2)_b-O-(CH_2)_c$, wherein a is 3, 4, or 5 and b is one, 2 or 3, and c is one, 2, or 3, with the proviso that b plus c is 2, 3, or 4, with the further proviso that $R_{12}$ and $R_{13}$ may be the same or different, and wherein $R_{14}$ is hydrogen or phenyl.

When the blocking group $R_{18}$ is tetrahydropyranyl, the tetrahydropyranyl ether derivative of any hydroxy moieties of the CBA-type intermediates herein is obtained by reaction of the hydroxy-containing compound with 2,3-dihydropyran in an inert solvent, e.g., dichloromethane, in the presence of an acid condensing agent such as p-toluenesulfonic acid or pyridine hydrochloride. The dihydropyran is used in large stoichiometric excess, preferably 4 to 100 times the stoichiometric amount. The reaction is normally complete in less than an hour at 20-50° C.

When the protective group is tetrahydrofuranyl, 2,3-dihydrofuran is used, as described in the preceding paragraph, in place of the 2,3-dihydropyran.

When the protective group is of the formula $-C(OR_{11})(R_{12})-CH-(R_{13})(R_{14})$, wherein $R_{11}$, $R_{12}$, $R_{13}$, and $R_{14}$ are as defined above; a vinyl ether or an unsaturated cyclic or heterocyclic compound, e.g.,

1-cyclohexen-1-yl methyl ether, or 5,6-dihydro-4-methoxy-2H-pyran is employed. See C.B. Reese, et al., Journal of the Chemical Society 86, 3366 (1967). The reaction conditions for such vinyl ethers and unsaturated compounds are similar to those for dihydropyran above.

The protective groups as defined by $R_{18}$ are removed by mild acidic hydrolysis. For example, by reaction with (1) hydrochloric acid in methanol; (2) a mixture of acetic acid, water, and tetrahydrofuran, or (3) aqueous citric acid or aqueous phosphoric acid in tetrahydrofuran, at temperatures below 55° C, hydrolysis of the blocking group is achieved.

The protecting group $R_{18}$ also can be a benzylic group. This protecting group is well known in the art. For example, benzylation of alcohols with benzyl bromide and siliver oxide in DMF gives benzyl ether [E. Reinefeld and K.D. Heincke, Ber., 104, 265 (1971)] in excellent yield. The removal of benzyl group is easily carried out by catalytic hydrogenation.

Certain compounds of the present invention are preferred. Thus, compounds of the formula I wherein $Y_1$ is a valence bond, n is zero, $X_1$ is -O-, -S-, or -NH-, m is 1, and $Q_1$ is $-CO_2R_1$ are preferred. More preferred in this class of compounds are those compounds wherein $R_1$ is hydrogen or methyl and $R_2$ is hydrogen, hydroxy, methoxy, or acetoxy; wherein $R_1$ is hydrogen or methyl, and $R_2$ is fluoro or trifluoromethyl; wherein $R_1$ is a pharmacologically acceptable cation selected from the group consisting of sodium, potassium or calcium and $R_2$ is hydrogen, hydroxy, methyl or acetoxy; or wherein $R_1$ is a pharmacologically acceptable cation selected from sodium, potassium, or calcium and $R_2$ is fluoro or trifluoromethyl.

Another preferred class of compounds are those wherein $Y_1$ is -O-; m is 1; $X_1$ is -O-, -S-, or -NH-, and $Q_1$ is $-CO_2R_1$. More preferred in this latter class of compounds are those wherein $R_1$ is hydrogen or methyl and $R_2$ is hydrogen, hydroxy, methoxy, or acetoxy; wherein $R_1$ is hydrogen or methyl, and $R_2$ is fluoro or trifluoromethyl; wherein $R_1$ is a pharmacologically acceptable cation selected from the group consisting of sodium, potassium or calcium and $R_2$ is hydrogen, hydroxy, methyl or acetoxy; or wherein $R_1$ is a pharmacologically acceptable cation selected from sodium, potassium, or calcium and $R_2$ is fluoro or trifluoromethyl.

Sodium 2-hydroxy-5-[N-(3-pyridinyl)aminomethyl]benzoate; sodium

2-methoxy-5-[N-(3-pyridinyl)aminomethyl]benzoate; and 4-[[(3-pyridinylaminomethyl]phenoxy]-acetic acid, sodium salt are the most preferred compounds of the present invention.

DESCRIPTION OF THE PREFERRED EMBODIMENTS

The present invention is seen more fully by the Examples given below.

Example 1: 4-[(3-Pyridinylamino)methyl]benzoic Acid, Methyl Ester (Formula I: $X_1$ is -NH-, m is 1, $R_2$ is hydrogen, $Y_1$ is a valence bond attached para to $-X_1-(CH_2)_m-$, m is zero, and $Q_1$ is $-CO_2CH_3$)

Refer to Chart A (conversion of A-1 and A-2 to A-4).

4-Carboxybenzaldehyde is treated with excess diazomethane-ether and the solvent is removed in vacuo to give a pale yellow solid. This solid is used without purification. A round-bottomed flask equipped with a magnetic stirring bar and a reflux condenser is charged with 3.1 g (33 mmol) 3-aminopyridine, 4.92 g (30 mmol) 4-carboxybenzaldehyde methyl ester and 60 mL toluene. The mixture is heated at 120°C under a nitrogen atmosphere for 24 h. Toluene is removed under reduced pressure to give a deep brown oil. This oil is then dissolved in 60 ml methanol and the solution is cooled to -10°C under a nitrogen atmosphere. Sodium borohydride powder (2.28 g, 60 mmol) is added over a period of 5 min. The mixture is stirred at about -10 to -5°C for 30 min. Saturated ammonium chloride is then added to the mixture and methanol is removed under reduced pressure. The concentrate is treated with saturated sodium bicarbonate and is extracted with ethyl acetate. The organic phase is washed with water, brine, and dried over anhydrous magnesium sulfate. Crystallization from hexane-ethyl acetate gives a light yellow solid. The mother liquor is subjected to liquid chromatographic purification. The product is chromatographed using 166 g silica gel-60 (40-63μ), eluting with methylene chloride-acetone (1:1), and taking 40 mL fraction. Fractions (26-40) are homogenous by thin-layer chromatography (TLC) (same Rf as the yellow solid) and are combined and concentrated in vacuo to give a solid. This solid and the yellow solid are combined and recrystallized from ethyl acetate-hexane to give a 2.99 g (41%) of a white solid with a mp of 123°C. The NMR ($CDCL_3$, TMS, δ) spectrum reveals peaks at 8.18-6.72, 4.40 and 3.88. The infrared (IR) spectrum (νmax) yields peaks at 3240, 3050, 3035, 2980, 2950, 2925, 2870, 2850,

1725, 1720, 1590, 1530, 1470, 1440, 1420, 1350, 1310, 1300, 1280, 1260, 1110, 1020, 770, and 710 $cm^{-1}$. The high resolution mass spectrum yields an ion at m/e 242.1072. The carbon:hydrogen:nitrogen (C:H:N) ratio is 69.03; 6.08; 11.27.

Example 2: 4-[(3-Pyridinylamino)methyl]benzoic Acid, Sodium Salt (sodium salt of Example 1)

A round-bottomed flask equipped with a magnetic stirring bar is charged with 1.21 g (5.0 mmol) amino-ester of Example 1, 5.25 mL 1N sodium hydroxide, and 15.75 mL methanol. The mixture is stirred at room temperature under a nitrogen atmosphere for 48 h. The solvent is removed in vacuo to give a light yellow solid (1.2 g). TLC in methylene chloride-acetone (1:1) shows one spot at the origin.

Preparation 1: 4-(Formyl)-phenoxy-acetic Acid, Methyl Ester

Refer to Chart B (conversion of B-1 to B-2).

A round-bottomed flask equipped with a magnetic stirring bar is charged with 12.2 g (0.1 mol) of 4-hydroxybenzaldehyde, 30.6 g (0.2 mol) of bromoacetate, 16.5 g (0.12 mol) potassium carbonate, and 200 mL glyme. The mixture (a brown solution suspended with white powder) is stirred at room temperature under a nitrogen atmosphere for 4 h. TLC shows no starting material remaining. The mixture is neutralized with cold 10% sodium bisulfate, extracted twice with ether (1L). The ether layer is washed with water, saturated sodium bicarbonate, and brine, and drived over anhydrous magnesium sulfate. Filtration and concentration give a yellow oil. Crystallization twice from ethyl acetate-hexane affords 14.6 g (75.3% yield) of a pale yellow solid with a mp of 43.5-44.5°C. The NMR ($CDCL_3$, TMS, $\delta$) spectrum reveals peaks at 9.90, 7.92, 7.76, 7.10, 6.96, 4.74, and 3.78. The IR ($\nu$max) spectrum reveals peaks at 2730, 1770, 1750, 1700, 1685, 1605, 1585, 1510, 1230, 1210, 1165, 1085, and 820 $cm^{-1}$. High resolution mass spectrum reveals an ion at 194.0579. The C:H ratio is 61.96; 5.19.

Example 3: [4-[(3-Pyridinylamino)methyl]phenoxy]-acetic Acid Methyl Ester and 2-[4-[(3-Pyridinylamino)methyl]phenoxy]-ethanol (Formula I: $X_1$ is NH, m is 1, $R_2$ is hydrogen, $Y_1$ is -O- and is para to $-X_1-(CH_2)_m-$, n is 1, and $Q_1$ is $-CO_2CH_3$ or $-CH_2OH$)

Refer to Chart B (conversion of B-2 to B-4 and B-5).

A round-bottomed flask equipped with a magnetic stirring bar and a reflux condenser is charged with 2.25 g (24.0 mmol), 3-aminopyridine, 3.88 g (20 mmol) the aldehyde-ester of Preparation 1, and 60 mL toluene. The mixture is heated at 120°C for 5 h. The condenser is replaced by a distillation head and the toluene is removed by distillation (the bath temperature is raised to 140°C). The residue (a deep brown oil) is then cooled to 0-5°C while dissolving in 40 mL methanol. Then sodium borohydride powder (1.52 g, 40 mmol) is added slowly over a period of 10 min. The mixture ( a light brown solution) is stirred for an additional 20 min. This solution is then poured into ice-aqueous ammonium chloride and the methanol is removed under reduced pressure. The residue is extracted with warm ethyl acetate. The organic phase is washed with water, brine, and dried over anhydrous magnesium sulfate. Activated charcoal is added to remove color. Filtration through celite gives a light pink colored solution. LC, using 324 g silica gel-60 (40-63μ), eluting with ethyl acetate-hexane-ethanol (10:1:1), taking 50 mL fractions, affords a less polar component (fr. 23-36) which is crystallized from ethyl acetate-hexane to give the amino-ester (mp. 101-2°C, 1.7 g, 31% yield). The more polar component (fr. 41-63) which is crystallized from ethyl acetate-hexane to give the amino-alcohol as a white solid (m.p. 110-111° C, 1.33 g, 24.6%). The spectral data is as follows:

For the less polar amino-ester: The NMR ($CDCL_3$, TMS, δ) spectrum reveals peaks at 8.18-7.88, 7.50-6.74, 4.62, 4.28, and 3.78. The IR (νmax) spectrum reveals peaks at 3230, 3150, 3090, 3060, 1740, 1610, 1590, 1580, 1510, 1310, 1290, 1270, 1215, 1180, 1000, and 805 $cm^{-1}$. The high resolution mass spectrum reveals an ion at 272.1148. The C:H:N ratio is 66.24; 5.96; 10.21.

For the more polar amino-alcohol: NMR (Acetone-$d_6$, TMS, δ) spectrum reveals peaks at 8.16-6.74 and 4.38-3.64. The IR (νmax) spectrum reveals peaks at 3280, 3160, 1615, 1595, 1590, 1580, 1530, 1510, 1485, 1320, 1250, 1170, 1080, 1060, 920, 815, 790, and 705 $cm^{-1}$. The high resolution mass spectrum reveals an ion at m/e 244.1213.

<u>Example 4</u>: 4-[(3-Pyridinylamino)methyl]phenoxy]-acetic Acid, Sodium Salt (sodium salt of Example 3)

A round-bottomed flask equipped with a magnetic stirring bar is charged with 816 mg (3.0 mmol) the amino-ester of Example 3, 3.3 ml of 1N sodium hydroxide and 9.9 ml of methanol. The mixture is stirred at room temperature under a nitrogen atmosphere for 24 h. The solvent is removed in vacuo to give a white powder (800 mg). TLC in ethyl

acetate-hexane-ethanol (10:1:1) shows only one spot at the origin.

Preparation 2: Methyl 5-Formyl-2-methoxy-benzoate and Methyl-5-Formyl-2-hydroxy-benzoate

Refer to Chart A (Prepration of A-2).

A round-bottomed flask equipped with a magnetic stirring bar is charged with 4.5 g (27.1 mmol) of the 5-formyl salicyclic acid (Aldrich Chemical Co.). Freshly prepared diazomethane in ether is added until TLC analysis shows no starting material remaining. Ether is removed under reduced pressure and the resulting oil is subjected to liquid chromatography using 324 g of HPLC grade silica gel, eluting with chloroform (fractions 1-50), chloroform-ethanol (40:1) (fractions 51-100), and collecting 40 ml fractions. Fractions 17-50, homogeneous by TLC, are combined and concentrated in vacuo to give a white solid (1.5 g, 30%). Crystallizatin from ethyl acetate-hexane gives the 2-hydroxy compound as colorless needles, mp. 80-81°C. Fractions 73-110, homogeneous by TLC, are combined and concentrted in vacuo to give a pale yellow solid (1.0 g, 19%). Cystallization from ethyl acetate-hexane affords the 2-methoxy compound, mp. 83-84°C. When the starting material is dissolved in EtOAc-$CH_3OH$ (1:1) and treated with excess diazomethane, very little formation of the 2-methoxy compound is observed and the 2-hydroxy compound is obtained in better than 90% yield without chromatography. The yield of the 2-methoxy compound is only about 5%. Spectral data is as follows:

For the 2-methoxy compound:

The NMR ($CDCl_3$, TMS, $\delta$) spectrum reveals peaks at 10.0, 8.36-7.05, 4.02, and 3.94. The infrared (Nujol) spectrum reveals peaks at 2955, 2925, 2870, 2850, 1710, 1705, 1690, 1670, 1610, 1500, 1460, 1440, 1430, 1380, 1320, 1300, 1275, 1260, 1210, 1190, 1150, 1010, 820, 810, and 790 $cm^{-1}$. The mass spectrum yields an ion at m/e 194.0583. The C:H ratio is 61.50; 5.10.

For the 2-hydroxy compound:

The NMR ($CDCL_3$, TMS, $\delta$) spectrum reveals peaks at 9.98, 8.46-7.05, and 4.02. The infrared (Nujol) spectrum reveals peaks at 3065, 3010, 2950, 2930, 2870, 1620, 1590, 1490, 1450, 1390, 1380, 1350, 1345, 1300, 1270, 1230, 1210, 1160, 1090, 840, 795, and 720 $cm^{-1}$. The mass spectrum yields an ion at m/e 180.0419. The C:H ratio is 59.66; 4.51.

Example 5: Methyl 2-Methoxy-5-[N-(3-pyridinyl)aminomethyl]Benzoate

(Formula I: $X_1$ is -NH-, m is 1, $R_2$ is $-OCH_3$ and is para to $-X_1-(CH_2)_m-$, $Y_1$ is a valence bond and is meta to $-X_1-(CH_2)_m-$, n is zero, and $Q_1$ is $-CO_2CH_3$)

Refer to Chart A (conversion of A-2 to A-4).

A round-bottomed flask equipped with a magnetic stirring bar, a condenser and a Dean-Stark moisture receiver is charged with 536.4 mg (5.7 mmol) of 3-aminopyridine, 1.0 g (5.2 mmol) of methyl 5-formyl-2-methoxy-benzoate, 49.5 mg (0.26 mmol, 5 mol%) of p-toluene sulfonic acid, and 8.2 mL of benzene. The mixture is heated to reflux (bath temperature 110°C) and stirred for 16 h. Benzene is then removed under reduced pressure. The brown concentrate is dissolved in 20.8 mL of methanol and the solution is cooled to -20°C under a nitrogen atmosphere. To this solution 395.2 mg (10.4 mmol) of sodium borohydride powder is added over a period of 5 min. The mixture is stirred at the same temperature for an additional 30 min. The reaction is quenched with saturated aqueous ammonium chloride and the methanol is removed under reduced pressure. The concentrate is extracted twice with chloroform (500 mL). The organic layer is washed with water, brine, and dried over anhydrous magnesium sulfate. Filtration and concentration afford a light brown oil. Liquid chromatography is carried out using 166 g of HPLC grade silica gel, eluting with chloroform-ethanol (20:1), and collecting 40 mL fractions. Fractions 6-25 are homogeneous by TLC and are combined and concentrted in vacuo to give a white solid. Crystallization from hexane-ethyl acetate afford pure titled crystals, 1.05 g, 75% yield, with a mp of 132-3°C. The NMR ($CDCL_3$, TMS, $\delta$) spectrum reveals peaks at 8.22-6.80, 4.32 and 3.90. The infrared (Nujol) spectrum reveals peaks at 3240, 3030, 3010, 1700, 1620, 1590, 1580, 1500, 1350, 1290, 1260, 1210, 1000, and 810 $cm^{-1}$. The mass spectrum yields an ion at m/e 272.1161. The C:H:N ratio is 65.81:5.97:10.14.

Example 6: Sodium 2-Methoxy-5-[N-(3-pyridinyl)aminomethyl]benzoate (sodium salt of Example 5)

A round-bottomed flask equipped with a magnetic stirring bar is charged with 544.6 mg (2.0 mmol) of methyl 2-methoxy-5-[N-(3-pyridinyl)aminomethyl]benzoate (Example 5), 2.7 mL of 1N sodium hydroxide and 8.4 mL of methanol. The mixture is stirred under a nitrogen atmosphere at room temperature for 5 days. The mixture is then lyophilized to give a white solid. Crystallization from acetone-water affords the titled product (500 mg, mp 240-8°C, decomposition). The NMR spectrum indicates the sodium salt. TLC also shows only one spot on the origin in chloroform-ethanol (20:1).

Example 7: Methyl 2-Hydroxy-5-[N-(3-pyridinyl)aminomethyl]benzoate (Formula I: $X_1$ is -NH-, m is 1, $R_2$ is -OH and is para to $X_1$-$(CH_2)_m$, $Y_1$ is a valence bond and is meta to -$X_1$-$(CH_2)_m$-, n is zero, and $Q_1$ is $-CO_2CH_3$)

Refer to Chart A (conversion of A-2 to A-4).

A round-bottomed flask equipped with a magnetic stirring bar, a condenser, and a Dean-Stark moisture receiver is charged with 5.2 g (55 mmol) of 3-aminopyridine, 9.0 g (50 mmol) of methyl 2-hydroxy-5-formyl-benzoate (Preparation 2), 0.475 g (5 mol%) of p-toluene sulfonic acid, and 250 mL of benzene. The mixture is heated to reflux (bath temperature 110°C) and stirred for 3 h. A small amount of a purple precipitate appears. TLC shows no starting material remaining. Benzene is removed under reduced pressure to give a brown solid. This solid is dissolved in 400 mL of methanol and the solution is cooled to between -15 and -10°C under a nitrogen atmosphere. Sodium borohydride powder (5.7 g, 0.15 mol) is added slowly over a period of 10 min. The mixture is then stirred at this temperature for an additional 30 min. TLC shows the reaction to be completed. The mixture is slowly poured into a mixture containing 100 mL of saturated aqueous ammonium chloride and crushed ice. The methanol is removed under reduced pressure. The residue is treated with 50 mL of 1N sodium hydroxide and extracted twice with ethyl acetate (1 L). The organic layer is washed with brine and dried over anhydrous magnesium sulfate. Filtration and concentration afford a brown oil. Liquid chromatography is carried out using HPLC grade silica gel (490 g), eluting with chloroform-ethanol (40:1), and collecting 40 mL fractions. Fractions 43-65 are homogeneous on TLC and are combined and concentrated in vacuo to give pure titled product (11.7 g, 90%). Crystallization from hexane-ethyl acetate affords an off-white solid with a mp 77-8°C. The NMR ($CDCL_3$, TMS, $\delta$) spectrum reveals peaks at 8.22-6.70, 4.32 and 3.94. The infrared (Nujol) spectrum reveals peaks at 3230, 3040, 2985, 2950, 1690, 1580, 1490, 1420, 1345, 1335, 1325, 1315, 1295, 1210, 1190, 1180, 1085, 800, and 790 $cm^{-1}$. The mass spectrum yields an ion at m/e 258.1020. The C:H:N ratio is 65.10:5.59:10.83.

Example 8: Sodium 2-Hydroxy-5-[N-(3-pyridinyl)aminomethyl]benzoate (sodium salt of Example 7)

A round-bottomed flask equipped with a magnetic stirring bar is charged with 1.03 g (4.0 mmol) of methyl 2-hydroxy-5-[N-(3-pyridinyl)aminomethyl]benzoate (Example 7), 4.2 mL of 1N NaOH and 8.4 mL of methanol. After stirring the solution under a nitrogen atmosphere for 24 h, TLC shows less than 50% completion. Another portion of 1N NaOH (4.2 mL) is added and the resulting mixture is stirred for 72 h. Methanol is removed under reduced pressure and the residue is crystallized from acetone-water to give an off-white solid (the titled product), 1.02 g, mp 220-240°C, decomp. Infrared (Nujol, nmax) shows peaks at 3310, 1670, 1635, and 1580 $cm^{-1}$.

Example 9: 3-[N-(2'-methoxycarbonylmethoxy)benzyl]-aminopyridine and 3-[N-(2'-hydroxyethoxy)benzyl]-aminopyridine (Formula I: $X_1$ is -NH-, m is 1, $Y_1$ is -O- and is ortho to $-X_1-(CH_2)_m-$, $R_2$ is hydrogen, n is 1, and $Q_1$ is $-CO_2CH_3$ or $-CH_2OH$)

Refer to Chart B (conversion of B-2 to B-4 and B-5).

Part A: 2-Formylphenoxyacetic acid (3.14 g, 17.4 mmol) (Aldrich Chemical Co.) is suspended in approximately 50 mL of ethyl acetate and treated with excess ethereal diazomethane. All of the previously undissolved solid gradually dissolves and concentration under reduced pressure gives the crude methyl ester as a dark brown oil. The NMR ($CDCl_3$, TMS, $\delta$) spectrum reveals peaks at 10.63, 7.97-6.80, 4.78, and 3.77. The infrared (film, $\nu$max) reveals peaks at 2950, 2850, 1745, 1675, 1595, 1485, 1430, 1390, 1280, 1200, 1105, 1065, 835, 760, 710, and 6.45 $cm^{-1}$. The crude methyl ester is used in Part B without further purification.

Part B: The crude methyl ester is dissolved in 100 mL of benzene and the 3-aminopyridine (1.93 g, 20.5 mmol) and p-toluenesulfonic acid (0.20 g, 1.1 mmol) are added. The reaction flask is fitted with a Dean-Stark trap and condenser and the magnetically stirred mixture is heated to reflux for 4 h. After this time some unreacted methyl ester is still visible by TLC and an additional 0.1 g of p-toluenesulfonic acid is added. Continued reflux for another 18 h shows little change in the progress of the reaction. The reaction mixture is let cool and filtered through a pad of celite. The filtrate is concentrated under reduced pressure to give the crude imine product as a dark brown oil.

Part C: This oil is dissolved in 50 mL of methanol and cooled to -20°C under a nitrogen atmosphere. Sodium borohydride (1.2 g, 32 mmol) is added in portions over 2-3 min and the resulting mixture is stirred for 45 min at which time TLC analysis indicates the reduction is complete. Saturated aqueous ammonium chloride (25 mL) is added to quench the reaction. Methanol is removed under reduced pressure and the concentrate is diluted with 50 mL of saturated aqueous sodium bicarbonate. This mixture is extracted with 200 mL of methylene chloride and the organic phase is washed with 100 mL of brine, dried ($MgSO_4$), filtered and concentrated to give 5.75 g of the crude product mixture as a dark brown oil. This material is chromatographed in 194 g of HPLC grade silica gel, eluting with hexane-ethyl acetate-ethanol (5:5:1), collecting 40 ml fractions. Fractions 16-25 are homogeneous by TLC and are combined and concentrated to give 2.17 g (46%) of the titled ester. This material solidifies and is recrystallized from ethyl acetate/hexane to give a pale yellow granular solid with a mp of 76-78°C. Similarly fractions 31-54 afforded 2.06 g (48%) of the alcohol which later solidifies. Recrystallization from ethyl acetate/hexane affords the products as pale yellow needles with a mp of 117-118°C. Characterization data is as follows:

For the ester: The NMR ($CDCl_3$, TMS, $\delta$) spectrum peaks are observed at 8.20-6.73, 4.72, 4.43, and 3.80. The infrared (Njuol, $\nu$max) spectrum peaks are observed at 3390, 1740, 1601, 1586, 1577, 1513, 1492, 1483, 1435, 1404, 1310, 1285, 1255, 1240, 1213, 1177, 1164, 1112, 1084, 1069, 976, 910, 812, 794, 757, and 628 $cm^{-1}$. The mass spectrum reveals ions at m/e 272.1170, 244, 213, 199, 198, 179, 151, 133, 121, 107, 91, and 78. The C:H:N ratio is 65.77:6.13:10.12.

For the alcohol: The NMR ($d_4$-MeOH, TMS, $\delta$) spectrum reveals peaks at 8.03-6.77, 4.82, 4.38, and 4.21-3.80. The infrared (Nujol, $\nu$max) spectrum reveals peaks at 3319, 3110, 1598, 1583, 1528, 1489, 1404, 1358, 1317, 1278, 1254, 1238, 1111, 1090, 1048, 1015, 927, 903, 791, 748, 704, 635, and 622 $cm^{-1}$. The mass spectrum reveals ions at m/e 244.1220, 227, 214, 199, 181, 151, 133, and 107. The C:H:N ratio is 68.27:6.62:11.43.

Example 10: 2-[(3'-pyridinyl)aminomethyl]-phenoxy Acetic Acid, Sodium Salt (sodium salt of B-4 compound of Example 9)

The starting ester of Example 9 (1.91 g, 7.02 mmol) is dissolved in 25 mL of methanol. Sodium hydroxide reagent (1.0 N, 7.40 mL, 7.4

mmol) is added and the resulting solution is stirred at room temperature for 24 h at which time TLC confirms the hydrolysis is complete. Methanol is removed under reduced pressure and the concentrate is diluted with 50 mL of water. The aqueous phase is washed with 25 mL of ethyl acetate (to decolorize the pale yellow solution). The organic phase is discarded. The mixture is filtered through a cotton plug, frozen, and lyophilized to give 1.97 g of the titled product as a white fluffy powder.

Example 11: 3-[N-(3'-Methoxycarbonylmethoxy)benzyl]-aminopyridine and 3-[N-(3'-hydroxyethoxy)benzyl111]-aminopyridine (Formula I: $X_1$ is -NH-, m is 1, $Y_1$ is -O- and is meta to $-X_1-(CH_2)_m-$, $R_2$ is hydrogen, n is 1, $Q_1$ is $-CO_2CH_3$ or $-CH_2OH$)

Refer to Chart B (conversion of B-2 to B-4 and B-5).

Part A: 3-Formylphenoxyacetic acid (5.0 g, 28 mmol) (Pfaltz and Bauer, Inc.) is suspended in approximately 50 ml of ethyl acetate and treated with excess ethereal diazamethone. All of the undissolved solid gradually dissolves and concentrations under reduced pressure gives the crude methyl ester as an orange-colored oil. The NMR ($CDCl_3$, TMS, δ) spectrum reveals peaks at 10.02, 7.63-7.15, 4.72, and 3.80. The infrared (film, νmax) reveals peaks at 2950, 2830, 2730, 1755, 1695, 1590, 1485, 1440, 1390, 1210 (broad), 1170, 1150, 1075, 1000, 910, 790, 745, 685 and 650 $cm^{-1}$. The crude methyl ester is used in Part B without further purification.

Part B: The crude methyl ester is dissolved in 150 mL of benzene and the 3-aminopyridine (3.1 g, 33 mmol) and p-toluenesulfonic acid (0.32 g, 1.7 mmol) are added. The flask is fitted with a Dean-Stark trap and condenser and the magnetically stirred mixture is heated to reflux for 4 h. After this time some unreacted methyl ester is still visible by TLC and an additional 1.0 g of 3-aminopyridine is added. Continued reflux for 1 h shows little change in the course of the reaction. The reaction mixture is cooled, filtered through a celite pad washing with additional benzene. The combined filtrates are concentrated to give the crude imine product as a red oil.

Part C: This oil is dissolved in 80 mL of methanol and cooled to -25°C under a nitrogen atmosphere. Sodium borohydride (1.9 g, 50 mmol) is added in portions over 3 min. The resulting mixture is stirred at -25°C for 20 min at which time 30 mL of saturated aqueous

ammonium chloride is added to quench the reaction. Methanol is removed under reduced pressure and the concentrte is diluted with 50 mL of saturated aqueous sodium bicarbonate and 50 mL of water and extracted with 300 mL of ethyl acetate. The organic phase is washed with 100 mL of brine, dried ($MgSO_4$), filtered and concentrated to give the crude product mixture as a dark-brown oil. This material is chromatographed in 194 g of HPLC grade silica gel, eluting with hexane-ethyl acetate-ethanol (5:5:1) and collecting 40 mL fractions. Fractions 7-17 afford impure ester while fractions 18-36 give the alcohol contaminated with impurities. The impure ester (fractions 7-17) is rechromatographed on 194 g of HPLC grade silica gel, eluting with methylene chloride-acetone (3:1) and collecting 30 mL fractions. Fractions 12-40 are homogeneous by TLC and are combined and concentrated to give 6.01 g (79%) of the ester as a pale yellow oil. In a similar fashion the impure alcohol (fractions 18-36) is rechromatographed on 54 g of HPLC grade silica gel, eluting with hexane-acetone (1:1) and collecting 30 mL fractions. Fractions 21-43 are homogeneous by TLC and are combined and concentrated to give 0.98 g (14%) of pure alcohol. This material solidifies and is recrystallized from chloroform/hexane to give buff-colored crystals with a mp of 94-95°C. Characterization data is as follows:

For the ester: The NMR ($CDCl_3$, TMS, $\delta$) spectrum reveals peaks at 8.17-6.70, 5.20, 4.57, 4.23, and 3.68. The infrared (film, $\nu$max) spectrum reveals peaks at 3398, 3264, 3035, 2953, 2851, 1758, 1590, 1486, 1438, 1418, 1376, 1302, 1213, 1158, 1088, 1010, 890, 849, 794, 709, and 691 $cm^{-1}$. The mass spectrum yields ions at m/e 272.1166, 213, 179, 121, 107, 91, and 78.

For the alcohol: The NMR ($CDCl_3$, TMS, $\delta$) spectrum reveals peaks at 8.13-6.71, 5.58, and 4.14-3.74. The infrared (Nujol, $\nu$max) spectrum reveals peaks at 3316, 1593, 1525, 1490, 1440, 1357, 1324, 1275, 1248, 1153, 1133, 1092, 1082, 1060, 1046, 1012, 951, 906, 871, 773, 705, 695, and 634 $cm^{-1}$. The mass spectrum yields ions at m/e 244.1220, 213, 199, 184, 169, 151, 133, 107, 91, and 79. The CH:H:N ratio is 68.52:6.83:11.47.

<u>Example 12</u>: 3-[(3'-pyridinyl)aminomethyl]-phenoxy Acetic Acid, Sodium Salt (sodium salt of the B-4 compound of Example 11)

The starting ester (1.16 g. 4.26 mmol) of Example 11 is dissolved

in 15 mL of methanol. Sodium hydroxide reagent (1.0 N, 4.25 mL, 4.25 mmol) is added and the resulting solution is stirred at room temperature for 17 hrs at which time TLC analyses confirms the hydrolysis is complete. Methanol is removed under reduced pressure and the concentrate is diluted with water. The aqueous phase is washed with 50 mL of ethyl acetate-ether (1:1) in an attempt to decolorize the pale yellow solution. The organic layer is discarded. The mixture is filtered through a cotton plus and lyophilized to give 1.25 g of titled product as a light-brown granular solid.

<u>Preparation 3</u> 4-Carboxybenzyl alcohol, Methyl Ester

Refer to Chart A (conversion of A-2 to A-6).

The 4-carboxybenzaldehyde methyl ester of Example 1 is reacted with two equivalents of sodium borohydride in methanol at -10 ± -5° C for 30 minutes. The reaction mixture is worked up as described in Example 1 to give the titled product.

<u>Preparation 4</u> 4-Carboxybenzyl chloride, Methyl Ester

Refer to Chart A (conversion of A-6 to A-7).

The 4-carboxybenzyl alcohol, methyl ester of Preparation 3 is treated with triphenylphosphine-carbon tetrachloride [I.M. Downie, J.B. Holmes, and J.B. Lee, Chem. Ind. 900 (1966)] to give the titled product by means well known in the art.

<u>Example 13</u> 4-[(3-Pyridinyloxa)methyl]benzoic Acid, Methyl Ester (Formula I: $X_1$ is -O-, m is 1, $R_2$ is hydrogen, $Y_1$ is a valence bond attached para to $-X_1-(CH_2)_m-$, m is zero, and $Q_1$ is $-CO_2CH_3$)

Refer to Chart A, (conversion of A-7 to A-8).

A round-bottomed flask equipped with a magnetic stirring bar and a gas inlet tube is charged with 528 mg (11.0 mmol) of sodium hydride (50% in oil dispersion) under a nitrogen atmosphere. The hydride is washed twice with dry hexane and the powder is suspended in 20 ml of DMF. A solution of 3-hydroxy-pyridine (951 mg, 10 mmol, Aldrich Chemical Co.) in 4 ml of DMF is added dropwise over 5 min at room temperature. The resulting mixture is stirred at room temperature for one hour. The mixture is then cooled to 0-5° C with an ice-water bath and a solution of 4-carboxybenzyl chloride methyl ester from Preparation 4 (1.84 g, 10 mmol) in 4 ml of DMF is added dropwise over 5 min. The reaction is monitored by thin-layer chromatography (TLC). When the reaction is completed, the reaction mixture is poured into

brine with crushed ice. The resulting mixture is extracted with ethyl acetate. The organic layer is washed with brine and dried over anhydrous magnesium sulfate. Filtration and concentration is followed by chromatography to give the titled product.

Example 14 4-[(3-Pyridinylthio)methyl]benzoic Acid, Methyl Ester (formula I: $X_1$ is -S-, m is 1, $R_2$ is hydrogen, $Y_1$ is a valence bond attached para to $-X_1-(CH_2)_m-$, n is zero, and $Q_1$ is $-CO_2CH_3$)

Refer to Chart A (conversion of A-7 to A-8).

Using the procedure described in Example 13, and replacing 3-hydroxy pyridine with 3-mercarpto pyridine (H. Fuerst, M. Hetzig, and W. Goebel, J. prakt. Chem., 36, 160 (1967)) the titled product is obtained. Alternatively, 3-mercaptopyridine is stirred with the chloride of Preparation 4 in the presence of potassium carbonate in THF or glyme, yielding the titled product.

Preparation 5 3-Hydroxymethyl-phenoxyacetic Acid, Methyl Ester

Refer to Chart B (conversion of B-2 to B-6).

The 3-formylphenoxy acetic acid methyl ester of Example 11 is treated with two equivalents of sodium borohydride in methanol at about -20 to -10° C for 30 minutes to give the titled product. The product is purified either by crystallization or chromatography.

Preparation 6 3-Chloromethyl-phenoxyacetic Acid, Methyl Ester

Refer to Chart B (conversion of B-6 to B-7).

The product of Preparation 5 is treated with triphenylphosphine-carbon tetrachloride as in Preparation 4 to give the titled product.

Example 15 [3-(Pyridinylthiomethyl)phenoxy]-acetic Acid, Methyl Ester (Formula I: $X_1$ is -S-, m is one, $R_2$ is hydrogen, $Y_1$ is -O- and is meta to $-X_1-(CH_2)_m-$

Refer to Chart B (conversion of B-7 to B-8).

Alkylation of the anion of 3-mercaptopyridine with 3-chloromethyl-phenoxyacetic acid, methyl ester (Preparation 6), using the procedure of Example 14, gives the titled product.

Preparation 7 t-Butyldimethylsilyloxy Methyl-4-methylthio-benzene

Refer to Chart C (conversion of C-1 to C-2).

A round-bottomed flask equipped with a magnetic stirring bar is charged with 15.4 g (0.1 mol) of p-(methylthio)-benzyl alcohol (Aldrich Chemical Co.), 18 g (0.12 mol) of t-butyldimethylsilyl

chloride (Aldrich Chemical Co.), 16.3 g (0.24 mol) of imidazole, and 100 ml of DMF. The resulting mixture is stirred at room temperature for 16 hr. TLC is used to monitor the reaction. The mixture is then quenched with 10 ml of water at 0-5° C and extracted with 1 L of Skellysolve B (a commercial mixture of essentially n-hexane). The organic layer is washed with water, 10% sodium bisulfate, saturated sodium bicarbonate, water and brine. It is then dried ($MgSO_4$), filtered, and concentrated. The crude product is purified either by chromatography or vacuum distillation.

Preparation 8 4-t-Butyldimethylsilyloxymethyl-thiobenzene

Refer to Chart C (conversion of C-2 to C-3).

The product of Preparation 7 is treated with chlorine in carbon tetrachloride followed by Amberlyst 15 Ion-exchange resins [J. M. Lavanish, Tetrahedron Lett., 3847 (1973)] to give the titled product.

Preparation 9 4-(t-Butyldimethylsilyloxy methyl)phenylthioacetic Acid, Methyl Ester

Refer to Chart C (conversion of C-3 to C-4).

The arylthiol of Preparation 8 is alkylated using the procedure described in Preparation 1, employing two equivalents of bromoacetate, 1.2 equivalents of potassium carbonate, and glyme as the solvent. The product is purified by chromatography or crystallization.

Preparation 10 4-Hydroxymethylphenylthio-acetic Acid, Methyl Ester

Refer to Chart C (conversion of C-4 to C-5).

The removal of the silyl protecting group from the product of Preparation 9, using two equivalents of tetra-n-butylammonium fluoride [E.J. Corey and A. Vankateswarlu, J. Am. Chem. Soc. 94, 6190 (1972)] gives the titled product.

Preparation 11 4-Chloromethylphenylthio-acetic Acid, Methyl Ester

Refer to Chart C (conversion C-5 to C-6).

Using the procedure described in Preparation 4, the compound of Preparation 10 is converted to the titled product.

Example 16 4-[(3-Pyridinyloxa)methyl]phenyl Thio-acetic Acid, Methyl Ester (Formula I: $X_1$ is -O-, m is 1, $R_2$ is hydrogen, $Y_1$ is -S-, n is 1, $Q_1$ is $-CO_2CH_3$)

Refer to Chart C (conversion of C-6 to C-7).

The product of Preparation 11 is treated with the anion of 3-hydroxypyridine, prepared according to the procedure described in

Example 13, to give the titled product. Purification is carried out either by crystallization or chromatography.

Preparation 12 3-(N-Formyl)aminopyridine

3-Aminopyridine is treated with acetic-formic anhydride [V.C. Mehlenbacher, Organic Analysis 1, 37 (1953); W. Stevens and A. VanEs, Rec. Trav. 83:1287, 1294 (1964)] to give the titled compound.

Example 17 4-[3-(N-Formyl)pyridinylaminomethyl]phenylthio-acetic Acid, Methyl Ester (Formula I: $X_1$ is -NCHO-, m is 1, $R_2$ is hydrogen, $Y_1$ is -S-, n is 1, $Q_1$ is $-CO_2CH_3$)

Refer to Chart C (conversion of C-6 to C-7).

Following the procedure of Example 13, the anion of 3-(N-formyl)aminopyridine (Preparation 12) is reacted with the chloride of Preparation 11 to give the titled product.

Preparation 13 3-(N-Methyl)aminopyridine

3-(N-Formyl)aminopyridine of Preparation 12 is treated with lithium aluminum hydride in tetrahydrofuran (THF) [C.F. Huebner, E.M. Donoghue, A. J. Plummer, and P.A. Furness, J. Med. Chem., 9, 830 (1966)] to yield the titled product.

Example 18 4-[3-(N-Methyl)pyridinylaminomethyl]phenylthio-acetic Acid, Methyl Ester (Formula I: $X_1$ is $-NCH_3-$, m is 1, $R_2$ is hydrogen, $Y_1$ is -S-, n is 1, $Q_1$ is $-CO_2CH_3$)

Refer to Chart C (conversion of C-6 to C-7).

Following the procedure of Example 13, the anion of 3-(N-methyl)aminopyridine (Preparation 13) is reacted with the chloride of Preparation 11 to give the titled product.

Preparation 14 4-Acetamidobenzylalcohol

Refer to Chart D (Preparation D-1).

4-Acetamidobenzaldehyde (Aldrich Chemical Co.) is treated with sodium borohydride in methanol at about -10 to -5° C, as described in Preparation 3 to give the titled product. Purification is carried out either by chromatography or crystallization.

Preparation 15 t-Butyldimethylsilyloxymethyl-4-acetamido benzene

Refer to Chart D (conversion of D-1 to D-2).

4-Acetamidobenzoyl alcohol (Preparation 14) is treated with a silylating agent, as described in Preparation 7, to give the titled product. Purification is carried out either by chromatography or crystallization.

Preparation 16 t-Butyldimethylsilyloxymethylphenyl-4-acetamido-N-

acetic Acid, Methyl Ester

Refer to Chart D (conversion of D-2 to D-3).

Following the procedure of Example 13, the anion of t-butyldimethylsilyloxymethyl-4-acetamidobenzene (Preparation 16) is reacted with bromoacetate to give the titled product.

Preparation 17 Hydroxymethylphenyl-4-acetamido-N-acetic Acid, Methyl Ester

Refer to Chart D (conversion of D-3 to D-4).

Following the procedure of Preparation 10, the product of Preparation 16 is treated with tetra-n-butylammonium fluoride in THF to remove the silyl protecting group. The purification is carried out by chromatography or crystallization to give the titled product.

Preparation 18 Chloromethylphenyl-4-acetamido-N-acetic Acid, Methyl Ester

Refer to Chart D (conversion of D-4 to D-5).

Following the procedure of Preparation 4, the product of Preparation 17 is treated with triphenylphosphine-carbon tetrachloride to give the titled product. The purification is carried out by chromatography or crystallization.

Example 19 4-[3-(Pyridinylthiomethyl)phenyl]-aza-acetic Acid, Methyl Ester (Formula I: $X_1$ is -S-, m is 1, $R_2$ is hydrogen, $Y_1$ is -NH-, n is 1, $Q_1$ is $-CO_2CH_3$)

Refer to Chart D (conversion of D-5 to D-6 to D-7).

Following the procedure of Example 14 the product of Preparation 18 is reacted with the anion of 3-mercaptopyridine to give the product of the formula D-6 wherein $X_1$ is -S-. The acetyl group attached to nitrogen is removed by treatment with hydrochloric acid in ethanol-water by means well known in the art [J.R. Johnson, et al., Org. Synth. Coll. Vol 1, 111 (1932)], to give the titled product.

FORMULAS

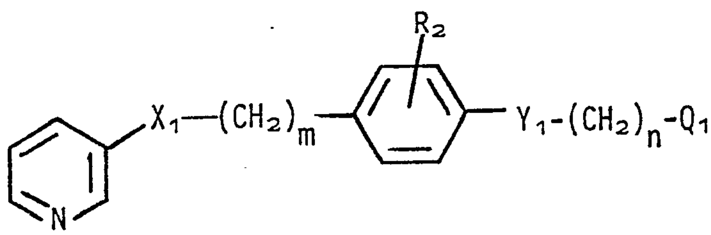

I

CHART A

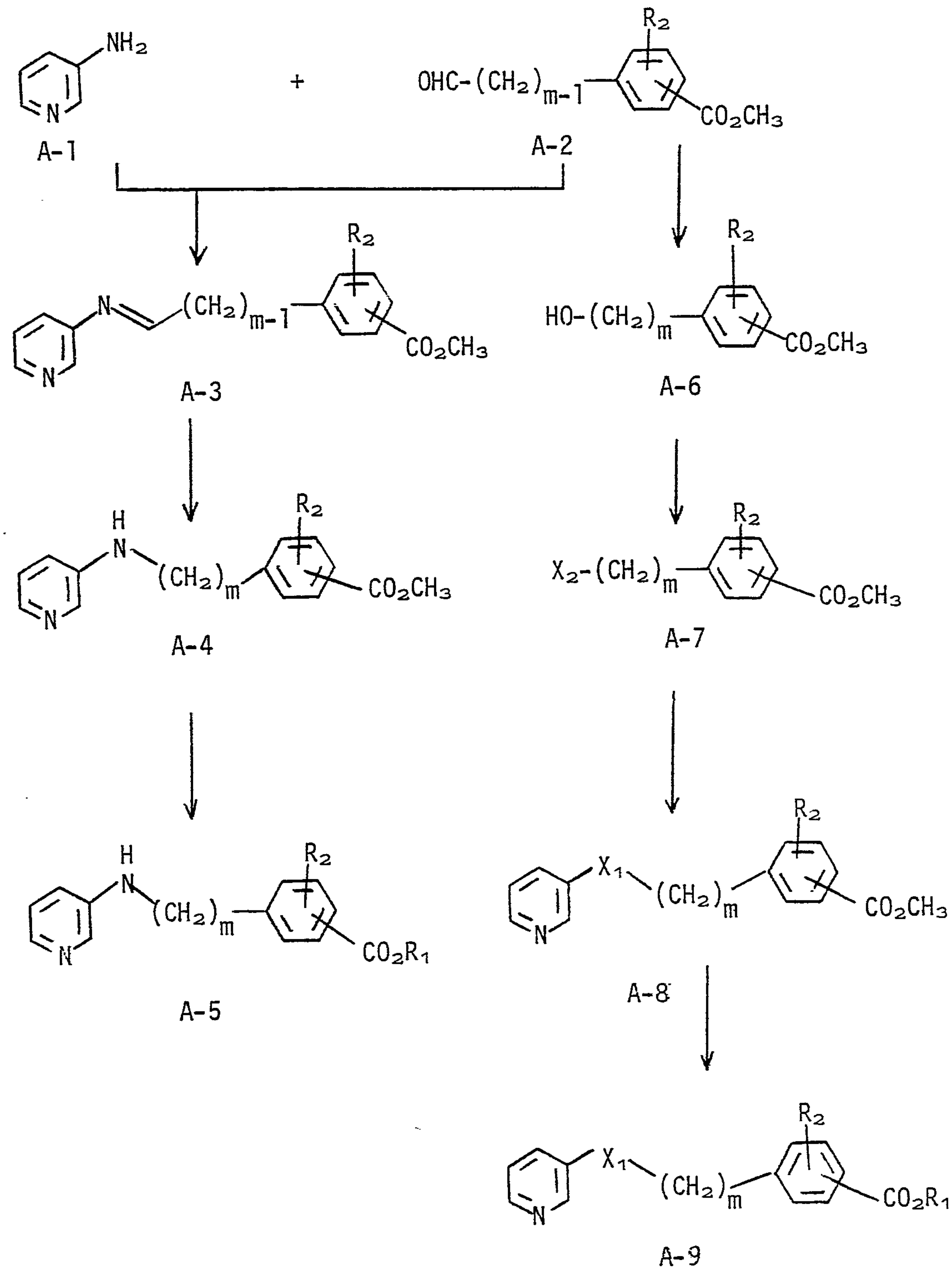

A-1

A-2

A-3

A-6

A-4

A-7

A-5

A-8

A-9

CHART B

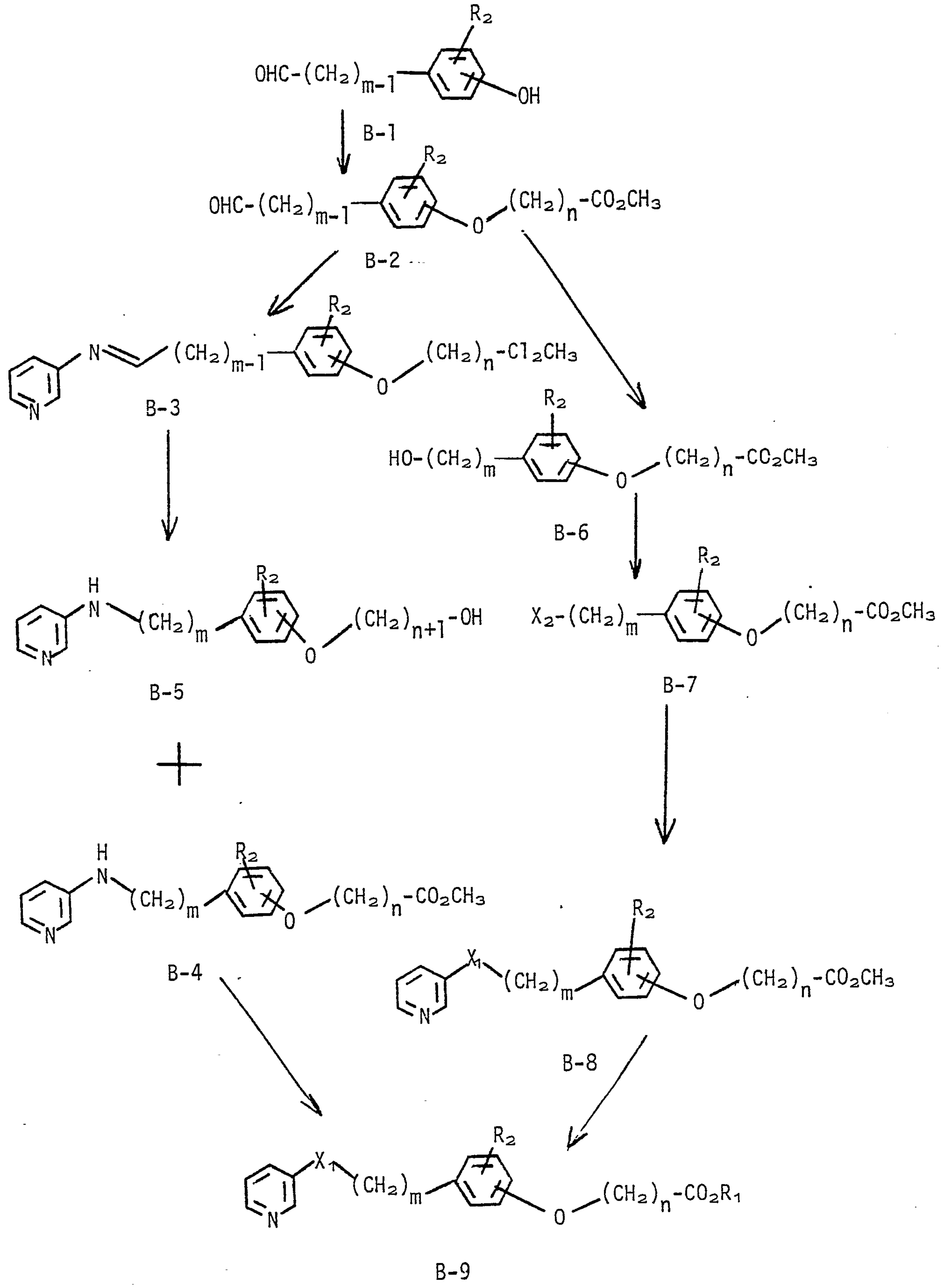

B-1

B-2

B-3

B-6

B-5

B-7

B-4

B-8

B-9

CHART C

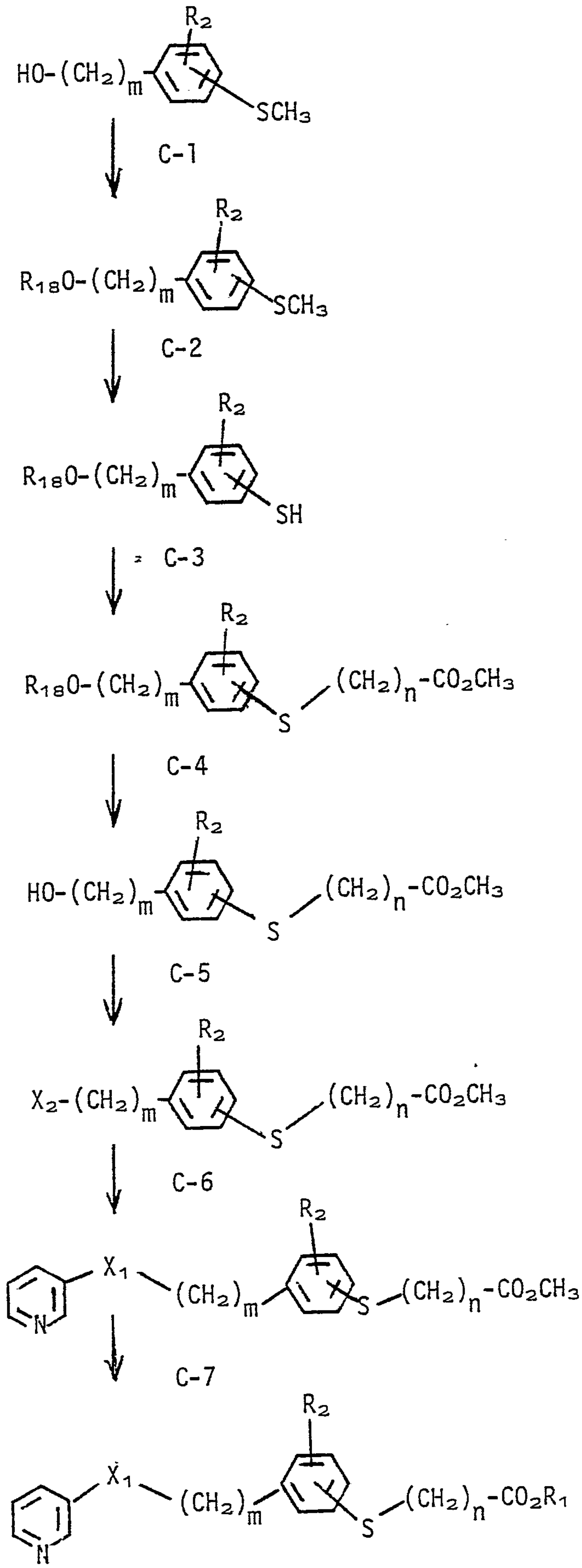

C-1

C-2

C-3

C-4

C-5

C-6

C-7

C-8

CHART D

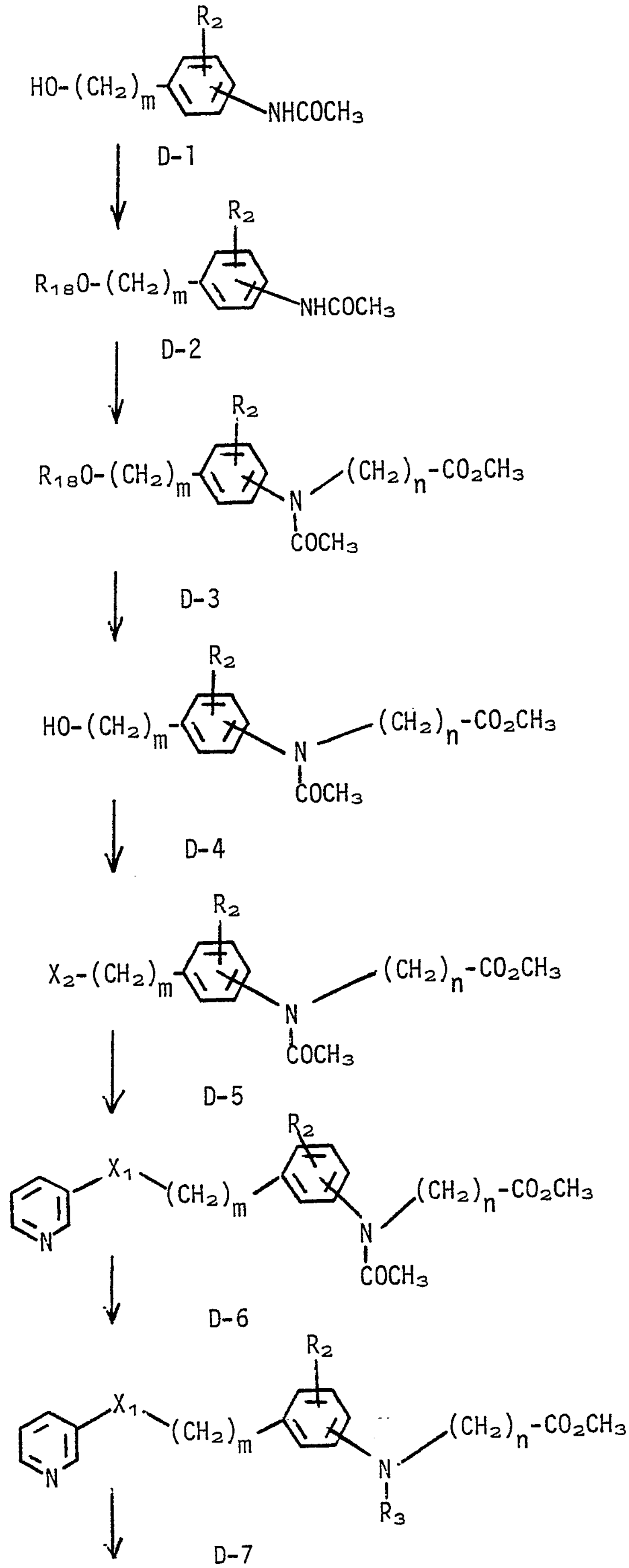

D-1

D-2

D-3

D-4

D-5

D-6

D-7

CHART D (continued)

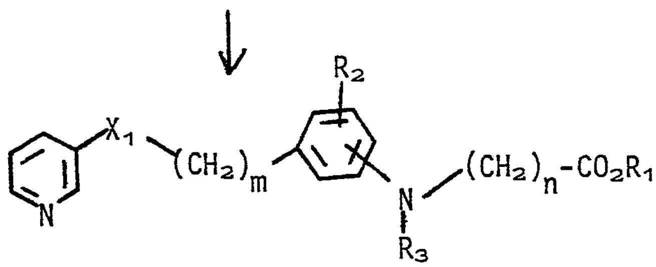

D-8

## CLAIMS

1. A compound of the Formula I,

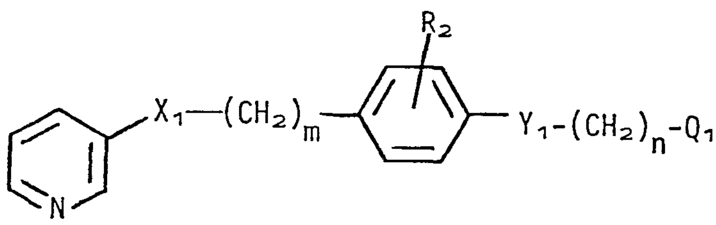

I

or a pharmacologically acceptable acid addition salt thereof, wherein $X_1$ is

(a) -O-,
(b) -S-, or
(c) $-NR_3-$;

wherein $Y_1$ is

(a) -O-,
(b) -S-,
(c) $-NR_3-$, or
(d) a valence bond;

wherein $R_2$ is

(a) hydrogen,
(b) hydroxy,
(c) methoxy,
(d) acetoxy,
(e) fluoro,
(f) chloro,
(g) bromo,
(h) methyl,
(i) trifluoromethyl,
(j) dimethylamino, or
(k) nitro;

wherein $Q_1$ is

(a) $-CO_2R_1$,
(b) $-CH_2OH$,
(c) -OH,
(d) -SH, or
(e) $-NR_3$;

wherein $R_1$ is

(a) hydrogen,

(b) a pharmacologically acceptable cation,

(c) $(C_1-C_{12})$ alkyl,

(d) $(C_3-C_{10})$ cycloalkyl,

(e) $(C_7-C_{12})$ aralkyl,

(f) phenyl,

(g) phenyl mono-, di-, or trisubstituted by chloro, $(C_1-C_3)$ or alkyl,

(h) or phenyl para-substituted by

(1) $-NHCO-R_{25}$,

(2) $-O-CO-R_{26}$,

(3) $-CO-R_{24}$,

(4) $-O-CO-(p-Ph)-R_{27}$, or

(5) $-CH=N-NH-CO-NH_2$;

wherein $R_{24}$ is phenyl or acetamidophenyl, $R_{25}$ is methyl, phenyl, acetamidophenyl, benzamidophenyl, or amino, $R_{26}$ is methyl, phenyl, amino or methoxy, $R_{27}$ is hydrogen or acetamido, and -(p-Ph) is 1,4-phenylene;

wherein $R_3$ is

(a) hydrogen,

(b) $(C_1-C_5)$alkyl,

(c) -CHO,

wherein m is an integer from one to 5;

wherein n is an integer from zero to 5, with the proviso that n is zero only when $Y_1$ is a valence bond and $Q_1$ is $-CO_2R_1$.

2. A compound of Claim 1, wherein $Y_1$ is a valence bond; n is zero; $X_1$ is -O-, -S-, or -NH-; m is 1; and $Q_1$ is $-CO_2R_1$.

3. A compound of Claim 1, wherein $Y_1$ is -O-; m is 1; $X_1$ is -O-, -S-, or -NH-; and $Q_1$ is $-CO_2R_1$.

4. A compound of Claim 2, wherein $R_1$ is hydrogen or methyl and $R_2$ is hydrogen, hydroxy, methoxy, or acetoxy.

5. A compound of Claim 3, wherein $R_1$ is hydrogen or methyl and $R_2$ is hydrogen, hydroxy, methoxy, or acetoxy.

6. A compound of Claim 2 selected from the group consisting of 4-[(3-Pyridinylamino)methyl]benzoic acid, methyl ester, and 4-[(3-Pyridinylamino)methyl]benzoic acid, sodium salt.

7. A compound of Claim 3 selected from the group consisting of [4-[(3-Pyridinylamino)methyl]phenoxy]-acetic acid methyl ester, and 4-[(3-Pyridinylamino)methyl]phenoxy]-acetic acid, sodium salt.

8. A compound of Claim 4 selected from the group consisting of:
Methyl 2-methoxy-5-[N-(3-pyridinyl)aminomethyl]benzoate,
Sodium 2-methoxy-5-[N-(3-pyridinyl)aminomethyl]benzoate,
Methyl 2-hydroxy-5-[N-(3-pyridinyl)aminomethyl]benzoate, and
Sodium 2-hydroxy-5-[N-(3-pyridinyl)aminomethyl]benzoate.

9. A compound of Claim 5 selected from the group consisting of:
3-[N-(2'-Methoxycarbonylmethoxy)benzyl]-aminopyridine,
2-[(3'-Pyridinyl)aminomethyl]-phenoxy acetic acid, sodium salt,
2-[N-(3'-Methoxycarbonylmethoxy)benzyl]-aminopyridine, and
3-[(3'-Pyridinyl)amonomethyl]-phenoxy acetic acid, sodium salt.

10. A compound of Claim 1 selected from the group consisting of 2-[4-[(3-Pyridinylamino)methyl]phenoxy]-ethanol, and 3-[N-(3'-Hydroxyethoxy)benzyl]-aminopyridine.